# EUROPÄISCHE PATENTSCHRIFT

(11) **EP 3 081 919 B1**
(45) Veröffentlichungstag und Bekanntmachung des Hinweises auf die Patenterteilung: **21.11.2018**
(21) Anmeldenummer: 15163863.2
(22) Anmeldetag: 16.04.2015
(51) Int. Cl.: G01N 3/40, G01N 3/08, G01N 33/15

(54) **PRÜFGERÄT ZUM DURCHFÜHREN VON HÄRTEMESSUNGEN**
TEST DEVICE FOR PERFORMING HARDNESS MEASUREMENTS
APPAREIL DE CONTRÔLE POUR METTRE EN OEUVRE DES MESURES DE DURETÉ

(43) Veröffentlichungstag der Anmeldung: 19.10.2016
(73) Patentinhaber: Kraemer, Thilo, 64291 Darmstadt (DE)
(72) Erfinder: Kraemer, Thilo, 64291 Darmstadt (DE)
(74) Vertreter: Vonnemann, Gerhard

(56) Entgegenhaltungen:
- EP-A1- 2 587 261
- WO-A1-98/53298
- WO-A1-2012/167289
- WO-A1-2015/039767
- CN-U- 204 177 690
- DE-A1-102004 036 777

## Beschreibung

Die Erfindung betrifft ein Prüfgerät zum Durchführen von Härtemessungen an Prüflingen umfassend eine Bruchkammer, die eine Festbacke und eine der Festbacke gegenüberliegende Pressbacke enthält.

Prüfgeräte zum Durchführen von Härtemessungen an Prüflingen, beispielsweise an Tabletten, sind bekannt. Dabei wird die Härtemessung in einer Bruchkammer durchgeführt, wobei auf einer ebenen Fläche zwischen einem beweglichen Stempel (Pressbacke) und einem fest angeordneten Teil (Festbacke) der Prüfling bis zum Bruch zerdrückt wird. Bei dieser Härtemessung wird der Wert der höchsten anzuwendenden Kraft ermittelt. Wichtig bei dieser Härtemessung ist die Ausrichtung des Prüflings, da der Prüfling in einer rechtwinkligen Achse zur Anlagefläche bzw. in einer Flucht mit der Krafteinleitung liegen muss. Längliche Prüflinge (zum Beispiel ein Oblong) können dabei beispielsweise mittels Hilfsmitteln, zum Beispiel mit Zangen, vor der Härtemessung ausgerichtet bzw. positioniert werden.

Nachteilig bei diesen Prüfgeräten mit solchen Hilfsmitteln ist, dass diese Geräte sehr komplex aufgebaut sind. Ferner kann durch das Halten der Prüflinge die Messung beeinflusst werden.

Ein Prüfgerät, bei dem die Positionierung eines Prüflings sehr schnell erfolgen kann, ist aus EP 1 717 415 A1 bekannt. EP 1 717 415 A1 beschreibt eine Härtemesseinrichtung zum Messen einer Härte eines Prüflings umfassend eine Bruchkammer, in der ein Boden von einem Teil eines Drehtellers gebildet wird, der um eine Drehachse gedreht werden kann. Auf dem Boden liegt der zu vermessende Prüfling. Durch Drehen des Drehtellers kann der Prüfling schnell in der Bruchkammer positioniert werden. Nach Positionieren des Prüflings kann die Härte gemessen werden. Längliche Prüflinge, zum Beispiel Oblongs, die keine parallelen Flanken besitzen, drehen dabei weg, so dass eine Messung nicht möglich ist. Das Prüfgerät eignet sich somit nicht zum Vermessen von Prüflingen mit komplexen Formen.

Auch aus WO 98/53298 A1 ist eine Vorrichtung zur Durchführung eines Härtetests an Prüfkörpern bekannt, mit einem Prüftisch zur Aufnahme des Prüfkörpers sowie einem Druckkolben und einem Gegenlager, wobei diese oberhalb des Prüftischs angeordnet und gegeneinander verfahrbar sind, wobei sich der Prüfkörper im Anwendungsfall zwischen Druckkolben und Gegenlager befindet und die beim Gegeneinanderdrücken von Druckkolben und Gegenlager aufgewendete Kraft oder eine dazu proportionale Größe mittels einer Kraftmessvorrichtung messbar und die Härte des Prüfkörpers daraus bestimmbar ist.

DE 10 2004 036 777 A1 beschreibt eine Vorrichtung zur Bruchfestigkeitsprüfung von Oblong-Tabletten, die eine horizontale Transportbahn, einen ortsfesten, als Anschlag für Oblong-Tablette dienenden Bruchbacken auf einer Seite der Transportbahn und einem mit einer Antriebs- und Messeinrichtung verbundenen, beweglichen Bruchbacken auf der anderen Seite der Transportbahn, sowie eine Einrichtung zum Ausrichten der Oblong-Tablette aufweist, die zwei gegenläufig angetriebene, nebeneinander angeordnete Walzen umfasst, deren Mantelflächen eine Aufnahme für die über die Transportbahn angelieferte Oblong-Tablette bilden, wobei die Walzen jeweils eine konische Mantelfläche mit einer oberen und einer unteren Mantellinie aufweisen, und dass die oberen Mantellinien der Walzen horizontal angeordnet sind und wobei die Achsen der Walzen entsprechend gegenüber der Horizontalen geneigt sind.

Des Weiteren ist aus EP 2 587 261 A1 eine Tablettenteststation bekannt, umfassend mindestens eine Aufnahme zur Übernahme von Tabletten von einem Auslass einer Beschickungsvorrichtung und mindestens ein Prüfmittel zum Prüfen, Testen und/oder Messen der Tabletten, wobei eine Hebevorrichtung zum Verfahren der Aufnahme relativ zur Beschickungseinrichtung vorgesehen ist.

Auch CN 204177690 U beschreibt eine Tablettentestvorrichtung die einen Träger, eine kraftaufnehmende Basis, eine Härteerfassungseinheit und einen Steuerkasten aufweist. Die kraftaufnehmende Basis und der Steuerkasten sind beide auf dem Träger angeordnet. Die Härteerfassungseinheit ist entsprechend oberhalb des Kraftaufnahmebodens angeordnet und umfasst einen Presskopf, eine Feder, einen Drucksensor, einen Zylinder und einen ersten Bildschirm.
Schließlich ist aus WO 2015/039767 A1 eine Härtemesseinrichtung zum Messen einer Härte eines Prüflings bekannt umfassend eine Bruchkammer, in der eine Festbacke vorgesehen ist, die gegenüber einer beweglichen Pressbacke angeordnet ist, wobei zumindest eine Anschlagleiste vorgesehen ist, die im Wesentlichen in einem rechten Winkel zu der Festbacke angeordnet ist, wobei ein Boden der Bruchkammer von einem Teil einer in beide Richtungen um eine Drehachse drehbaren ebenen Fläche gebildet wird, wobei auf dem Boden der Bruchkammer ein Prüfling anordenbar ist.

Aufgabe der vorliegenden Erfindung ist es deshalb, ein Prüfgerät zum Durchführen von Härtemessungen an Prüflingen bereitzustellen, mit dem auch Prüflinge, die komplexe Formen besitzen, vermessen werden können.

Diese Aufgabe wird durch ein Prüfgerät nach Anspruch 1 gelöst.
[A01] Die Erfindung betrifft somit ein Prüfgerät zum Durchführen von Härtemessungen an Prüflingen umfassend eine Bruchkammer, in der eine Festbacke und eine ihr gegenüberliegende bewegliche Pressbacke angeordnet ist. In der Bruchkammer ist ein Laufband zumindest teilweise angeordnet, wobei mit dem Laufband der Prüfling in der Bruchkammer einfach positionierbar ist. Vorteilhaft bei dem Prüfgerät ist, dass sowohl die Breitenmessungen als auch die Härtemessungen an Prüflingen mit sehr komplexen Formen durchgeführt werden können.
   Das Laufband ist als Endlosband ausgebildet. Ein Endlosband benötigt nur wenig Platz und läuft sehr stabil.
   Zumindest ein Teil dieses Laufbands bildet einen Boden der Bruchkammer. Dies hat den Vorteil, dass kein separater Boden vorgesehen werden muss. Vorteilhaft ist ferner, dass mit dem Laufband der Prüfling aus der Bruchkammer entfernt werden kann, nachdem der Härtetest durchgeführt worden ist.
   In dieser Bruchkammer ist ein Widerlager vorgesehen, das die Bruchkammer zu einer Seite hin begrenzt und sich von der Festbacke in Richtung der Pressbacke erstreckt, wobei das Widerlager im Wesentlichen in einem rechten Winkel zu der Festbacke und damit auch zu der Pressbacke angeordnet ist. Vorteilhaft bei diesem Widerlager ist, dass es nicht nur die Bruchkammer begrenzt, sondern gleichzeitig auch als Führung dient, entlang der der Prüfling mittels der Pressbacke in Richtung der Festbacke bewegt werden kann.
   Des Weiteren ist eine Verstellvorrichtung vorgesehen, auf der das Laufband angeordnet ist, wobei mittels der Verstellvorrichtung das Laufband in der Bruchkammer ausgerichtet werden kann. Dadurch ist es möglich, das Laufband in einem bestimmten Winkel in Bezug auf die Festbacke bzw. dem Widerlager anzuordnen. Dies hat den Vorteil, dass durch die Verstellung des Winkels des Laufbands auch Prüflinge mit komplexen Formen vermessen werden können. Die Verstellvorrichtung kann per Hand oder auch automatisch verstellt werden. Wird die Verstellvorrichtung automatisch verstellt, so ist dafür ein Antrieb vorgesehen, mit dem die Verstellvorrichtung bewegt wird.
[A02] Bevorzugt ist die Verstellvorrichtung als Plattenelement ausgebildet, da ein solches Plattenelement ein einfaches Bauelement ist.
[A03] In einer weiteren bevorzugten Ausführungsform ist unterhalb des als Endlosband ausgebildeten Laufbands eine Putzvorrichtung, zum Beispiel eine Bürste, vorgesehen, mit der das Endlosband von Resten des Prüflings befreit werden kann. Auf eine Putzvorrichtung innerhalb der Bruchkammer kann daher verzichtet werden.

Ausführungsbeispiele werden in den Figuren dargestellt und im Folgenden näher erläutert. Es zeigen:
- Figur 1: einen Ausschnitt eines Prüfgeräts mit einer Bruchkammer;
- Figur 2: eine weitere Ansicht des in Figur 1 gezeigten Prüfgeräts;
- Figur 3: eine weitere Ansicht des in Figur 1 gezeigten Prüfgeräts;
- Figur 4: eine weitere Ansicht des in Figur 1 gezeigten Prüfgeräts;
- Figur 5: eine weitere Ansicht des in Figur 1 gezeigten Prüfgeräts;
- Figur 6: eine weitere Ansicht des in Figur 1 gezeigten Prüfgeräts;
- Figur 7: eine weitere Ansicht des in Figur 1 gezeigten Prüfgeräts;
- Figur 8: eine weitere Ansicht des in Figur 1 gezeigten Prüfgeräts;
- Figur 9: eine weitere Ansicht des in Figur 1 gezeigten Prüfgeräts;
- Figur 10: eine weitere Ansicht des in Figur 1 gezeigten Prüfgeräts, wobei die Position des Laufbands verändert wurde;
- Figur 11: eine weitere Ansicht des in Figur 10 gezeigten Prüfgeräts;
- Figur 12: eine weitere Ansicht des in Figur 10 gezeigten Prüfgeräts;
- Figur 13: eine weitere Ansicht des in Figur 10 gezeigten Prüfgeräts;
- Figur 14: eine weitere Ansicht des in Figur 10 gezeigten Prüfgeräts;
- Figur 15: eine weitere Ansicht des in Figur 10 gezeigten Prüfgeräts;
- Figur 16: eine Variante eines Prüfgeräts, wobei ein Laufband auf einem Verstellelement angeordnet ist;
- Figur 17: eine weitere Ansicht des in Figur 16 gezeigten Prüfgeräts und
- Figur 18: eine schematische Darstellung des in Figur 16 gezeigten Prüfgeräts und
- Figur 19: eine weitere Ansicht des in Figur 10 gezeigten Prüfgeräts, wobei die Position des Laufbands verändert wurde.

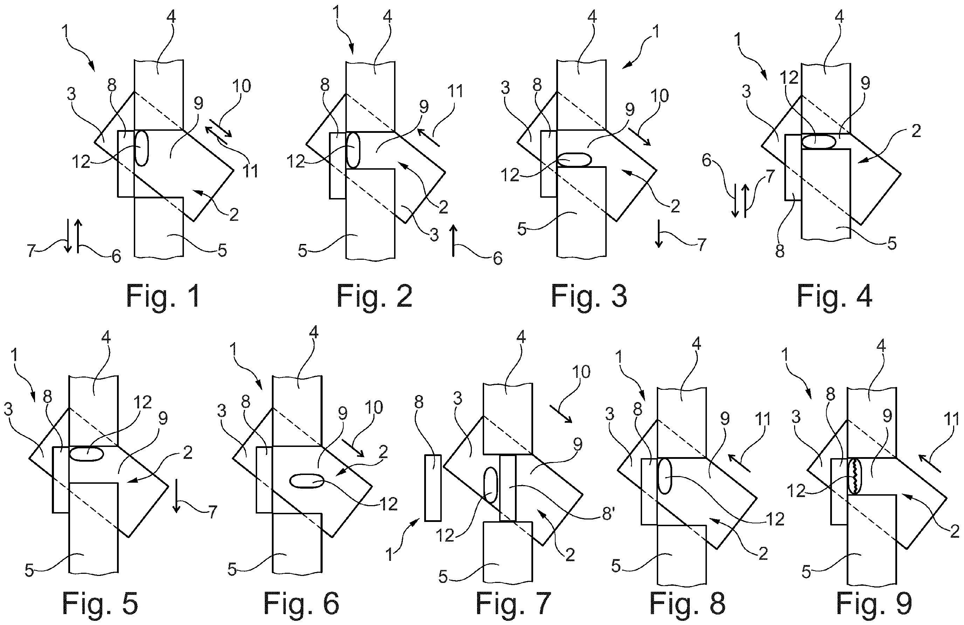

In Figur 1 ist ein Ausschnitt eines Prüfgeräts 1 zum Durchführen von Härtemessungen an Prüflingen dargestellt, das eine Bruchkammer 2 aufweist. In dem Prüfgerät 1 ist ein Laufband 3 vorgesehen, wobei das Laufband 3 zumindest teilweise in der Bruchkammer 2 angeordnet ist. In der Bruchkammer 2 ist eine fest angeordnete Festbacke 4 und eine ihr gegenüberliegende bewegliche Pressbacke 5 vorgesehen. Die Pressbacke 5 kann in Richtung der Festbacke 4 (Pfeil 6) hinbewegt oder von dieser fortbewegt werden (Pfeil 7). In der Bruchkammer 2 ist ein erstes Widerlager 8 vorgesehen ist, das die Bruchkammer 2 begrenzt und sich von der Festbacke 4 in Richtung der Pressbacke 5 erstreckt, wobei das Widerlager 8 im Wesentlichen in einem rechten Winkel zu der Festbacke 4 sowie auch zu der Pressbacke 5 angeordnet ist. Dieses Widerlager 8 ist ein Teil einer Transportvorrichtung, mit der ein Prüfling 12 zur Vermessung in die Bruchkammer 2 transportiert wird. Bei der Transportvorrichtung kann es sich beispielsweise um einen Transportstern oder einen Transportrechen handeln. Da solche Transportvorrichtungen bekannt sind, wir auf eine detaillierte Beschreibung verzichtet.
Das Laufband 3 ist unterhalb der Pressbacke 5, der Festbacke 4 sowie des Widerlagers 8 angeordnet und bildet zumindest teilweise einen Boden 9 der Bruchkammer 2. Vorzugsweise wird der gesamte Boden 9 durch das Laufband 3 gebildet. Das Laufband 3 ist als Endlosband ausgebildet und kann in Richtung des Pfeils 10 oder in die Richtung des Pfeils 11 bewegt werden. Ein solches Endlosband hat den Vorteil, dass es nur wenig Platz benötigt und sehr stabil läuft.
Auf dem Boden 9 der Bruchkammer 2 ist der Prüfling 12 aufgebracht worden, der vermessen werden soll. Dieser Prüfling 12 liegt mit einer Längsseite an dem Widerlager 8 und mit einem Ende an der Festbacke 4 an. Der Prüfling 12 ist damit in der Bruchkammer 2 in Längsrichtung ausgerichtet. Der Prüfling 12 ist in Figur 1 eine Tablette, die als Oblong ausgebildet ist. Es versteht sich, dass auch Prüflinge, die eine andere Form haben, mit dem Prüfgerät 1 vermessen werden können.

Dabei kann unterhalb des Laufbands 3 eine Waage (nicht zu sehen) angebracht sein, mit der die Masse des Prüflings 12 gemessen wird, sobald dieser auf das Laufband 3 aufgebracht worden ist. Unterhalb des als Endlosband ausgebildeten Laufbands 3 ist eine Putzvorrichtung (nicht zu erkennen), zum Beispiel eine Bürste, vorgesehen, mit der das Endlosband von Resten des Prüflings 12 befreit werden kann, nachdem der Härtetest durchgeführt wurde. Auf eine Putzvorrichtung innerhalb der Bruchkammer kann somit verzichtet werden.

Figur 2 zeigt eine weitere Ansicht des Ausschnitts des Prüfgeräts 1 gemäß Figur 1. Das Laufband 3 wird dabei in Richtung des Pfeils 11, das heißt in Richtung des Widerlagers 8, bewegt, so dass der Prüfling 12 ebenfalls in Richtung des Widerlagers 8 bewegt wird, so dass der Prüfling 12 schließlich mit seiner einen Längsseite fest an dem Widerlager 8 angeordnet ist. Die Pressbacke 5 ist bereits in Richtung der Festbacke 4, das heißt in Richtung des Pfeils 6, bewegt worden. Durch die Bewegung der Pressbacke 5 in Richtung der Festbacke 4 wird auch der Prüfling 12 in diese Richtung bewegt, bis er schließlich zwischen der Pressbacke 5 und der Festbacke 4 angeordnet ist. In dieser Position wird der Prüfling 12 gehalten und es wird die Länge des Prüflings 12 vermessen. Die Vermessung der Länge des Prüflings 12 kann beispielsweise mittels Kameras erfolgen, die jedoch in der Figur 2 nicht gezeigt sind.

Nach der Vermessung der Länge des Prüflings 12 wird die Pressbacke 5 wieder in die Ausgangsposition zurückgefahren und das Laufband 3 in Richtung des Pfeils 10 bewegt (Figur 3), bis der Prüfling mit einer Längsseite an der Pressbacke 5 anliegt (Figur 4). Es wird dann die Pressbacke 5 in Richtung des Pfeils 6, das heißt in Richtung der Festbacke 4 bewegt, so dass der Prüfling 12 zwischen der Festbacke 4 und Pressbacke 5 fixiert ist. In dieser Position wird die Breite des Prüflings 12 gemessen. Auch die Messung der Breite des Prüflings 12 kann mittels in Figur 4 nicht dargestellten Kameras erfolgen. Das Laufband 3 kann dabei angehalten werden, wie dies in Figur 4 der Fall ist.

Nach der Breitenmessung wird die Pressbacke 5 wieder in die Ausgangsposition zurückgefahren, das heißt die Pressbacke 5 wird in Richtung des Pfeils 7 bewegt (Figur 5). Das Laufband 3 steht währenddessen still. Anschließend wird das Laufband 3 in Richtung des Pfeils 10 bewegt, wodurch der Prüfling 12 von dem Widerlager 8 fortbewegt wird und der Prüfling 12 am äußeren Rand der Bruchkammer 2 angeordnet ist (Figur 6). Es wird dann die Transportvorrichtung bewegt, so dass ein zweites Widerlager 8' in die Bruchkammer 2 geführt wird. Dieses zweite Widerlager 8' bewegt den Prüfling 12 wieder zurück (Figur 7). Während der Prüfling 12 zurückbewegt und wieder in Längsrichtung ausgerichtet wird, steht das Laufband 3 still, wobei auch denkbar ist, dass das Laufband 3 währenddessen in Richtung des Pfeils 10 bewegt wird. Danach wird das zweite Widerlager 8' wieder aus der Bruchkammer 2 bewegt, so dass das erste Widerlager 8 wieder die Bruchkammer 2 begrenzt und sich von der Festbacke 4 in Richtung der Pressbacke 5 erstreckt. Das Widerlager 8 befindet sich somit wieder in der Ausgangsposition (Figur 8). Das Laufband 3 wird anschließend in Richtung des Pfeils 10 bewegt, wodurch der Prüfling 12 in Richtung des Widerlagers 8 geführt wird, bis er die in Figur 8 gezeigte Halteposition einnimmt. In dieser Halteposition ist der Prüfling 12 optimal für die anschließende Härtemessung positioniert.

Die Härtemessung ist in Figur 9 dargestellt. Dazu wird die Pressbacke 5 in Richtung der Festbacke 4 bewegt, bis die Pressbacke 5 mit dem Prüfling 12 in Kontakt steht, was durch den Pfeil 6 angedeutet ist. Dabei wird die Kraft, die auf den Prüfling 12 wirkt, schrittweise erhöht, bis der Prüfling 12 zerbricht. Dies geschieht dadurch, dass die Pressbacke 5 langsam in Richtung Festbacke 4, das heißt in Richtung des Pfeils 6, bewegt wird. Die Kraft, die zum Bruch des Prüflings 12 aufgewendet werden muss, ist als Härtewert definiert und wird über eine Kraftmessdose mit Dehnungsmessstreifen gemessen. Diese Kraftdose ist entweder in der Pressbacke 5 oder der Festbacke 4 angeordnet.

Nachdem die Härtemessung durchgeführt wurde, wird der zerbrochene Prüfling 12 aus der Bruchkammer 2 entfernt, indem das Laufband 3 in Richtung des Pfeils 10 bewegt wird.

Um das Laufband 3 von Resten des Prüflings 12 zu befreien, ist unterhalb des als Endlosband ausgebildeten Laufbands 3 eine Putzvorrichtung, zum Beispiel eine Bürste, vorgesehen, die jedoch in den Figuren 1 bis 9 aufgrund der Darstellung nicht zu sehen ist. Auf eine Putzvorrichtung innerhalb der Bruchkammer 2 kann daher verzichtet werden. Nachdem das Laufband 3 gereinigt wurde, kann ein weiterer Prüfling in die Bruchkammer 2 eingebracht werden.

In den Figuren 1 bis 9 wurde zuerst die Längenmessung (vergleiche Figur 2) und anschließend die Breitenmessung (vergleiche Figur 4) durchgeführt.

Es ist allerdings auch möglich, zuerst die Breitenmessung und danach die Längenmessung durchzuführen.

Da ein Prüfling, gerade im Falle eines Oblongs, mit einer Kante auf dem Boden 9 angeordnet sein kann, nachdem dieser in die Bruchkammer 2 eingebracht worden ist, ist es erforderlich, dass der Prüfling mit seiner Längsseite auf dem Boden 9 liegt. Nur wenn der Prüfling mit seiner Längsseite auf dem Boden 9 liegt, kann die Härtemessung nach dem Europäischen bzw. dem US-amerikanischen Arzneibuch durchgeführt werden. Zum Umwerfen des Prüflings kann an dem Widerlager 8 ein Vorsprung oder eine Nase vorgesehen sein (nicht dargestellt). Wird der Prüfling zu dem Widerlager 8 mit der Nase bzw. dem Vorsprung mittels des Förderbands 3 transportiert, so stößt der Prüfling an den Vorsprung bzw. die Nase des Wiederlagers 8. Dadurch wird der Prüfling umgeworfen, so dass der Prüfling anschließend mit seiner Längsseite auf dem Boden 9 der Bruchkammer 2 aufliegt.

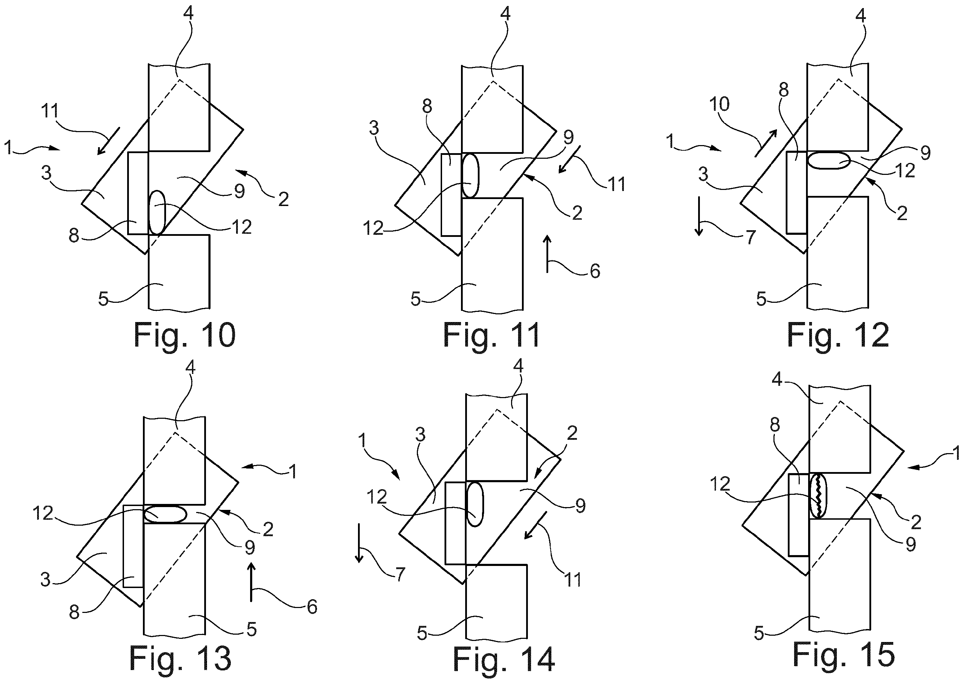

In den Figuren 10 bis 15 ist eine weitere Variante des Härtemessvefahrens gezeigt, das in der Bruchkammer 2 durchgeführt werden kann. Dazu ist das als Endlosband ausgestaltete Laufband 3 mittels einer Verstellvorrichtung um 90° gedreht worden. Die Verstellvorrichtung ist der Übersicht halber in den Figuren 10 bis 15 allerdings nicht gezeigt.

Nachdem der Prüfling 12 in die Bruchkammer 2 transportiert wurde, wird das Laufband 3 in Richtung des Pfeils 11 bewegt, so dass der Prüfling 12 in Richtung des Widerlagers 8 bewegt wird, bis der Prüfling 12 mit seiner einen Längsseite am Widerlager 8 und mit einem Ende an der Pressbacke 5 anliegt (Figur 10). In Figur 10 ist somit eine weitere Halteposition gezeigt. Anschließend wird die Pressbacke 5 in Richtung der Festbacke 4 bewegt (Pfeil 6). Dabei wird der Prüfling 12 entlang des Widerlagers 8 ebenfalls in Richtung der Festbacke 4 bewegt, bis dieser mit seinem anderen Ende an der Festbacke 4 anliegt (Figur 11). Hat der Prüfling 12 mit seinem anderen Ende die Festbacke 4 erreicht, so wird die Länge des Prüflings 12 gemessen. Das Laufband 3 kann während der Längenmessung weiter in Richtung des Pfeils 11 bewegt werden oder stillstehen. Die Pressbacke 5 wird etwas zurückbewegt, das heißt in Richtung des Pfeils 7 bewegt, damit der Prüfling 12 in der Bruchkammer 2 gedreht werden kann. Dazu wird das Laufband 3 in Richtung des Pfeils 10 bewegt, bis der Prüfling 12 mit einer Längsseite an der Festbacke 4 anliegt (Figur 12). Anschließend wird die Pressbacke 5 wieder in Richtung der Festbacke 4 bewegt, bis der Prüfling 12 zwischen der Festbacke 4 und der Pressbacke 5 eingeklemmt ist (Figur 13). Diese Bewegung der Pressbacke 5 ist durch den Pfeil 6 angedeutet. Es wird sodann die Breite des Prüflings 12 gemessen, wobei während der Messung das Laufband 3 stillstehen kann. Anschließend wird die Pressbacke 5 wieder in die Ausgangsposition zurück bewegt, das heißt in Richtung des Pfeils 7 bewegt. Zeitgleich wird das Laufband 3 in Richtung des Pfeils 11 bewegt wodurch sich der Prüfling 12 erneut in der Bruchkammer 2 dreht. Liegt der Prüfling 12 mit einer seiner Längsseiten an dem Widerlager 8 an, so wird die Härtemessung durchgeführt. Dazu wird die Pressbacke 5 erneut in Richtung der Festbacke 4 bewegt, bis der Prüfling 12 zwischen der Pressbacke 5 und der Festbacke 4 eingeklemmt ist. Die Kraft, die auf den Prüfling 12 wirkt, anschließend schrittweise erhöht, bis der Prüfling 12 zerbricht. Dies geschieht dadurch, dass die Pressbacke 5 langsam in Richtung Festbacke 4 bewegt wird. Diese Kraft wird mit einer Kraftdose gemessen, die entweder in der Pressbacke 5 oder in der Festbacke 4 angeordnet ist.
Nachdem die Härtemessung durchgeführt wurde, wird der zerbrochene Prüfling 12 aus der Bruchkammer 2 entfernt. Mittels der Putzvorrichtung, die unterhalb des als Endlosband ausgebildeten Laufbands 3 angeordnet ist, wird das Laufband 3 von Resten des zerbrochenen Prüflings 12 befreit.
Anschließend kann ein weiterer Prüfling, der vermessen werden soll, in die Bruchkammer 2 eingebracht werden.

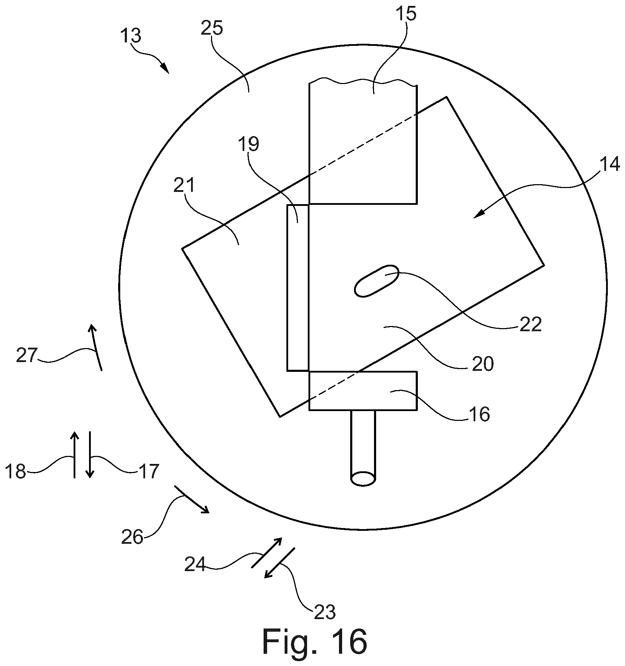

In Figur 16 ist eine erste Variante eines Prüfgeräts 13 dargestellt, wobei nur ein Ausschnitt dieses Prüfgeräts 13 gezeigt ist. Dieses Prüfgerät 13 umfasst eine Bruchkammer 14, die eine Festbacke 15 sowie eine ihr gegenüberliegende Pressbacke 16 aufweist. Wie auch die Pressbacke in den Figuren 1 bis 15 kann auch die Pressbacke 16 in Richtung der Festbacke 15 hin oder von dieser fortbewegt werden, was durch die Pfeile 17 und 18 angedeutet ist. In der Bruchkammer 14 ist ein Widerlager 19 vorgesehen, das die Bruchkammer 14 begrenzt und sich von der Festbacke 15 in Richtung der Pressbacke 16 erstreckt, wobei das Widerlager 19 im Wesentlichen in einem rechten Winkel zu der Festbacke 15 sowie auch zu der Pressbacke 16 angeordnet ist. Auch dieses Widerlager 19 ist Teil einer in Figur 16 nicht weiter dargestellten Transportvorrichtung.
Der Boden 20 der Bruchkammer 14 wird durch einen Abschnitt eines Laufbands 21 gebildet. Auf dem Laufband 21 liegt ein Prüfling 22, an dem die Längenmessung, die Breitenmessung und die Härtemessung durchgeführt werden soll. Bei diesem Prüfling 22 handelt es sich beispielsweise um eine Tablette, wobei der auf dem Laufband 21 liegende Prüfling 22 ein Oblong ist. Dieses Laufband 21 ist als Endlosband ausgebildet und kann in Richtung der Pfeile 23 und 24 bewegt werden. Dadurch kann auch der Prüfling 22 zu der Festbacke 15 oder dem Widerlager 19 bewegt werden. Insofern unterscheidet sich der Aufbau der Bruchkammer 14 nicht von der Bruchkammer 2 des Prüfgeräts 1, das in den Figuren 1 bis 15 dargestellt ist. Deshalb werden die Längenmessung, die Breitenmessung sowie die Härtemessung auch wie in den Figuren 10 bis 15 beschriebenen Verfahren durchgeführt.

Das Laufband 21 ist auf einer Verstellvorrichtung 25 angeordnet, wobei mittels der Verstellvorrichtung 25 die Ausrichtung des Laufbands 21 verändert werden kann, womit das Laufband 21 in der Bruchkammer 14 ausgerichtet wird. Vorzugsweise handelt es sich bei der Verstellvorrichtung 25 um ein Plattenelement und besonders bevorzugt um eine Scheibe. Mit der Verstellvorrichtung 25 kann das Laufband 21 in Richtung der Pfeile 26 bzw. 27 gedreht werden. Soll die Verstellvorrichtung 25 automatisch verstellt werden, so ist dazu ein Antrieb (nicht dargestellt) vorgesehen, mittels dem die Verstellvorrichtung 25 in Richtung des Pfeils 26 oder in Richtung des Pfeils 27 gedreht wird. Vorzugsweise handelt es sich bei dem Antrieb und einem Schrittmotor. Durch den Antrieb ist es möglich, dass das Laufband 25 um einen bestimmten Winkel, vorzugsweise um 45° gedreht werden kann. Durch diese horizontale Winkelverstellung des Laufbands 21 ist eine Anpassung an Prüflinge mit komplexen Formen möglich. In Figur 9 ist das Laufband 21 in Bezug auf das Widerlager um 45° gedreht. Diese Anordnung des Laufbands 21 ist vorteilhaft, wenn eine Längen- und eine Breitenmessung durchgeführt werden sollen. Dabei findet die Ausrichtung des Laufbands 21 statt, bevor an dem Prüfling 22 die entsprechenden Messungen vorgenommen werden. Zwischen den einzelnen Messungen wird die Position des Laufbands 21 also nicht verändert.

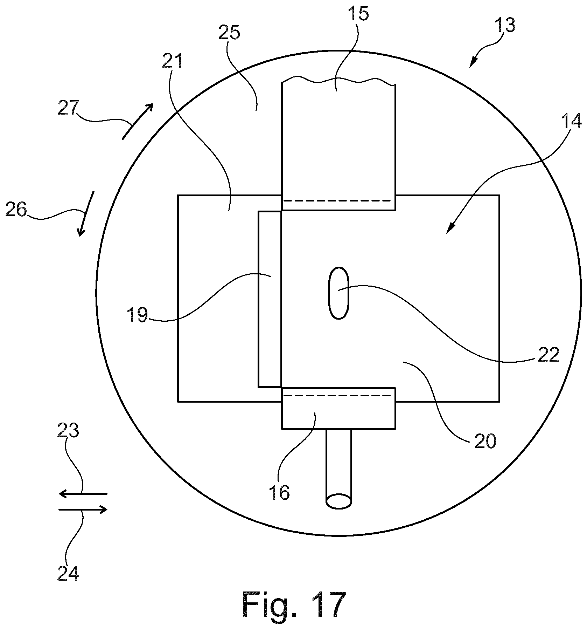

In Figur 17 ist eine weitere Ansicht des in Figur 9 gezeigten Ausschnitts des Prüfgeräts 13 dargestellt. In der Bruchkammer 14 liegt der Prüfling 22 auf dem Boden 20. Das Laufband 12 ist dabei gegenüber der in Figur 16 gezeigten Position um 45° gedreht, so dass sich die Laufrichtung des Laufbands 21 in Bezug auf das Widerlager 19 in einer 90°-Position befindet. Wird das Laufband 21 nun in Richtung des Pfeils 24 bewegt, so wird der Prüfling 22 aus der Bruchkammer 14 hinaus befördert, wohingegen der Prüfling 22 in Richtung des Widerlagers 19 befördert wird, wenn das Laufband 21 in Richtung des Pfeils 23 bewegt wird. Diese 90°-Position des Laufbands 21 eignet sich besonders gut, wenn der Prüfling 22 lediglich gehalten werden soll.

Auch das Widerlager 19 dieses Prüfgeräts 13 kann eine Nase oder einen Vorsprung zum Umwerfen eines Prüflings aufweisen, was jedoch in der Figur 17 nicht dargestellt ist.

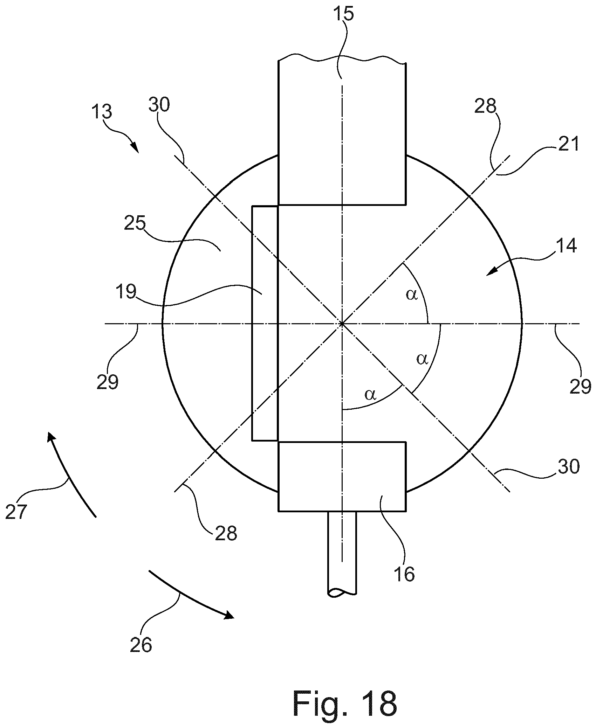

Figur 18 zeigt eine schematische Darstellung eines Ausschnitts des in Figur 16 gezeigten Prüfgeräts 13. Das Prüfgerät 13 besitzt die Bruchkammer 14 umfassend die Festbacke 15 sowie die ihr gegenüberliegende Pressbacke 16. Auch das Widerlager 19 ist zu erkennen. Auf dem Verstellelement 25 ist das Laufband 21 angeordnet, wobei in der Figur 18 die Positionen 28, 29, 30 des Laufbands 21 schematisch gezeigt sind.

Nimmt das Laufband 21 die Position 28 ein, so ist das Laufband 21 wie in Figur 16 gezeigt, in dem Prüfgerät 13 angeordnet. Wird das Laufband 21 um einen Winkel α in Richtung des Pfeils 27 gedreht, wobei α = 45° beträgt, so nimmt das Laufband 21 die Position 29 ein, die das Laufband 21 auch in Figur 17 einnimmt. Wird das Laufband 21 erneut um 45° gedreht, so nimmt das Laufband 21 die Position 30 ein. Durch erneute Drehung des Laufbands 21 um 45° wird eine 90°-Position erhalten.

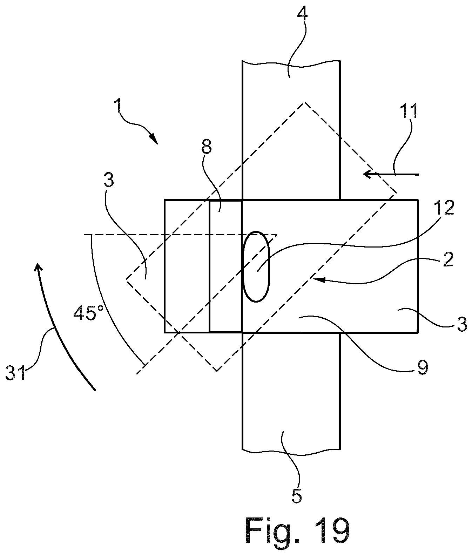

In Figur 19 ist eine weitere Ansicht des in Figur 10 gezeigten Ausschnitts des Prüfgeräts 1 dargestellt. Dabei ist eine Halteposition gezeigt, in der sich der Prüfling 12 befinden kann. Zu erkennen ist wiederum die Bruchkammer 2 mit der Pressbacke 5 und der Festbacke 4. In einem im Wesentlichen rechten Winkel zu der Festbacke 4 bzw. zu der Pressbacke 5 ist das Widerlager 8 der Transportvorrichtung angeordnet. Ein Teil des Laufbands 3 bildet wiederum den Boden 9 der Bruchkammer 2. Das Laufband 3 befindet sich allerdings in einer 90° Position in Bezug auf das Widerlager 8. Das Laufband 3 wurde somit um 45° gegenüber der in den Figuren 10 bis 15 gezeigten Position gedreht (Pfeil 31). Die in den Figuren 10 bis 15 gezeigte Position des Laufbands 3 ist durch gestrichelte Linien angedeutet.

### Bezugszeichenliste

- 1.: Prüfgerät
- 2.: Bruchkammer
- 3.: Laufband
- 4.: Festbacke
- 5.: Pressbacke
- 6.: Pfeil
- 7.: Pfeil
- 8.: Widerlager
- 9.: Boden
- 10.: Pfeil
- 11.: Pfeil
- 12.: Prüfling
- 13.: Prüfgerät
- 14.: Bruchkammer
- 15.: Festbacke
- 16.: Pressbacke
- 17.: Pfeil
- 18.: Pfeil
- 19.: Widerlager
- 20.: Boden
- 21.: Laufband
- 22.: Prüfling
- 23.: Pfeil
- 24.: Pfeil
- 25.: Verstellvorrichtung
- 26.: Pfeil
- 27.: Pfeil
- 28.: Position
- 29.: Position
- 30.: Position
- 31.: Pfeil

## Patentansprüche

1. Prüfgerät (1, 13) umfassend eine Bruchkammer (2, 14), die eine Festbacke (4, 15) und eine ihr gegenüberliegende Pressbacke (5, 16) enthält, wobei in der Bruchkammer (2, 14) ein Widerlager (8, 19) vorgesehen ist, das die Bruchkammer (2, 14) begrenzt und sich von der Festbacke (4, 15) in Richtung der Pressbacke (5, 16) erstreckt, wobei das Widerlager (8, 19) im Wesentlichen in einem rechten Winkel zu der Festbacke (4, 15) angeordnet ist, **dadurch gekennzeichnet, dass** in der Bruchkammer (2, 14) ein Endlosband (3, 21) zumindest teilweise angeordnet ist, wobei mit dem Endlosband (3, 21) ein Prüfling (12, 22) in der Bruchkammer (2, 14) positionierbar ist, wobei ein Teil des Endlosbands (3, 21) einen Boden (9, 20) der Bruchkammer (2, 14) bildet, wobei ein Antrieb vorgesehen ist, mit dem eine Verstellvorrichtung (25) drehbar ist, wobei auf der Verstellvorrichtung (25) das Endlosband (3, 21) angeordnet ist, so dass mittels der Verstellvorrichtung (25) das Endlosband (3, 21) in der Bruchkammer (2, 14) ausrichtbar ist.

2. Prüfgerät nach Patentanspruch 1, **dadurch gekennzeichnet, dass** die Verstellvorrichtung (25) ein Plattenelement ist.

3. Prüfgerät nach Patentanspruch 1, **dadurch gekennzeichnet, dass** unterhalb des Endlosbands (3, 21) eine Putzvorrichtung, vorgesehen ist, mit der das Endlosband (3, 21) von Resten des Prüflings (12, 22) befreit werden kann.

## Claims

1. Test device (1, 13) comprising a crush chamber (2, 14) that includes a fixed jaw (4, 15) and a pressing jaw (5, 16) opposite thereto, wherein in the crush chamber (2, 14) an abutment (8, 19) is provided that delimits the crush chamber (2, 14) and extends from the fixed jaw (4, 15) in the direction of the pressing jaw (5, 16), wherein the abutment (8, 19) is substantially disposed at a right angle to the fixed jaw (4, 15), **characterized in that** in the crush chamber (2, 14) a continuous feeder conveyance (3, 21) is at least partially disposed, wherein with the continuous feeder conveyance (3, 21) a test sample (12, 22) is positionable in the crush chamber (2, 14), wherein a portion of the continuous feeder conveyance (3, 21) forms a bottom (9, 20) of the crush chamber (2, 14), wherein a drive is provided with which an adjustment mechanism (25) is rotatable, wherein on the adjustment mechanism (25) the continuous feeder conveyance (3, 21) is disposed such that by means of the adjustment mechanism (25) the continuous feeder conveyance (25) can be aligned in the crush chamber (2, 14).

2. Test device as in patent claim 1, **characterized in that** the adjustment mechanism (25) is a plate element.

3. Test device as in patent claim 1, **characterized in that** underneath the continuous feeder conveyance (3, 21) a cleaning means is provided with which the continuous feeder conveyance (3, 21) can be cleaned of remnants of the test sample (12, 22).

## Revendications

1. Appareil de contrôle (1, 13), comprenant une chambre de rupture (2, 14) qui contient une mâchoire fixe (4, 15) et une mâchoire de pressage (5, 16) qui lui est opposée, un palier de butée (8, 19) se trouvant dans la chambre de rupture (2, 14), lequel délimite la chambre de rupture (2, 14) et s'étend depuis la mâchoire fixe (4, 15) en direction de la mâchoire de pressage (5, 16), le palier de butée (8, 19) étant disposé sensiblement selon un angle droit par rapport à la mâchoire fixe (4, 15), **caractérisé en ce qu'**une bande continue (3, 21) est au moins partiellement disposée dans la chambre de rupture (2, 14), un échantillon (12, 22) pouvant être positionné dans la chambre de rupture (2, 14) avec la bande continue (3, 21), une partie de la bande continue (3, 21) formant un fond (9, 20) de la chambre de rupture (2, 14), un mécanisme d'entraînement étant présent, lequel permet de faire tourner un dispositif de positionnement (25), la bande continue (3, 21) étant montée sur le dispositif de positionnement (25) de telle sorte que la bande continue (3, 21) peut être orientée dans la chambre de rupture (2, 14) au moyen du dispositif de positionnement (25).

2. Appareil de contrôle selon la revendication 1, **caractérisé en ce que** le dispositif de positionnement (25) est un élément en plaque.

3. Appareil de contrôle selon la revendication 1, **caractérisé en ce qu'**un dispositif de nettoyage se trouve au-dessous de la bande continue (3, 21), lequel permet de débarrasser la bande continue (3, 21) des restes de l'échantillon (12, 22).
